# EUROPÄISCHE PATENTSCHRIFT

(11) **EP 3 002 021 B1**
(45) Veröffentlichungstag und Bekanntmachung des Hinweises auf die Patenterteilung: **22.05.2019**
(21) Anmeldenummer: 15185439.5
(22) Anmeldetag: 16.09.2015
(51) Int. Cl.: A61M 5/50, A61M 35/00, A61M 5/34

(54) **STOPFEN ZUM AUFSETZEN AUF EIN ANSCHLUSSELEMENT EINER MEDIZINISCHEN SPRITZE**
PLUG FOR MOUNTING ON A CONNECTING ELEMENT OF A MEDICAL SYRINGE
BOUCHON A PLACER SUR UN ELEMENT DE RACCORDEMENT D'UNE SERINGUE MEDICALE

(30) Priorität: 02.10.2014 DE 102014114403
(43) Veröffentlichungstag der Anmeldung: 06.04.2016
(73) Patentinhaber: Gerresheimer Regensburg GmbH, 93047 Regensburg (DE)
(72) Erfinder: Fraas, Andreas Dipl. Ing., 92224 Amberg (DE)
(74) Vertreter: Hannke, Christian

(56) Entgegenhaltungen:
- EP-A1- 2 666 513
- WO-A1-2010/034470
- WO-A1-2012/024370
- WO-A2-03/076001
- IE-A1- 911 804
- US-A1- 2008 171 981
- US-A1- 2011 054 440

## Beschreibung

Die Erfindung betrifft einen Stopfen zum Aufsetzen auf ein Anschlusselement einer medizinischen Spritze nach dem Oberbegriff des Patentanspruchs 1.

Ein derartiger Stopfen ist beispielsweise der auf die Anmelderin zurückgehende TELC-Verschluss, der unter anderem unter der Internetadresse http://www.gerresheimer.com/uploads/tx qerinfo/12 telc en 01.pdf näher beschrieben ist. Dabei handelt es sich um einen originalitätsgesicherten Spritzenverschluss zum Aufdrehen. Ausgangspunkt der Erfindung ist hierbei, dass im täglichen Betrieb im Krankenhaus beziehungsweise einer medizinischen Einrichtung normale Glas- oder Kunststoffspritzen in vielfältiger Weise verwendet werden. Insbesondere für die Injektion von Wirkstoffen weisen die üblichen Standardspritzen einen Luer-Konus und gegebenenfalls einen Luer-Lock-Anschluss auf. An diese Standardanschlussstellen werden beispielsweise Kanülen zur intravenösen, trans- oder subkutanen Injektion von Wirkstoffen und Pharmazeutika beziehungsweise Nährstofflösungen angeschlossen. Dabei sind aber auch Schlauchverbindungen oder spezielle Konnektoren zum Anschluss an eine Standardspritze über die bekannten Luer-Systeme denkbar. Im normalen Krankenhausbetrieb werden derartige Standardspritzen vor allem aufgrund der leichten Verfügbarkeit aber auch zum Aufziehen, Abmessen und sogar zur oralen oder dermalen Applikation von Wirkstoffen verwendet.

Aus der Druckschrift DE 10 2011 111 552 A1 ist eine Spritze mit einem Verschluss bekannt. Der Verschluss umfasst eine das Nadelansatzstück der Spritze dichtend verschließende Verschlusskappe und eine Sicherungskappe, welche die Verschlusskappe umgreift und über einen Haltering an dem Nadelansatzstück befestigt ist. Ferner ist die Sicherungskappe und die Verschlusskappe einstückig ausgebildet.

Des Weiteren ist aus der DE 200 17 013 U1 ein Verschlusskörper für einen Luer-Lock-Anschluss bekannt. Der Verschlusskörper umfasst eine Kappe, welche den Luerkonus übergreift. An der Außenseite der Kappe ist ein Luerlockgewinde vorgesehen. Die Kappe ist innen konisch ausgebildet, so dass diese an dem Luerkonus anliegt, wobei der Innenkonus der Kappe sich über weniger als die Hälfte des der Länge des Luerkonus erstreckt

Diese allgemeine Verwendung von Standardspritzen birgt jedoch auch nicht unerhebliche Gefahren. So kommt es immer wieder vor, dass durch Unachtsamkeit, Zuständigkeitswechsel oder ähnliche Umstände eine zur oralen oder dermalen Verabreichung aufgezogene Spritze intravenös, trans- oder subkutan verabreicht wird. Eine derartige nicht zweckbestimmte Verwendung tritt insbesondere auch deshalb auf, da die vorhandenen Luer-Systeme dazu verleiten, eine Kanüle anzuschließen. Für das Aufziehen eines Wirkstoffes, eines Pharmazeutikums oder einer Ernährungslösung in eine derartige Spritze muss zuvor der Schutz beziehungsweise der Verschluss von der Spritze entfernt werden. Nach Entfernen dieses Verschlusses ist es möglich, den Wirkstoff, das Pharmazeutikum oder die Ernährungslösung in den Spritzenkörper aufzuziehen. Nach dem Aufziehen der Spritze würde man den Verschluss im normalen Betrieb nicht wieder aufsetzen, da man ihn bei der beispielsweise oralen Verabreichung wieder entfernen müsste. Zudem besteht hier wiederum die Gefahr, dass die Spritze nach erneutem Entfernen des Verschlusses mit einer Kanüle bestückt wird. In der näheren Vergangenheit ist es durch derartige Fehlverwendungen von Spritzen mit bereits aufgezogenen Wirkstoffen, Pharmazeutika oder Ernährungslösungen zu einigen Vorfällen mit zum Teil letalen Folgen für die Patienten gekommen.

Es ist daher Aufgabe der Erfindung einen Stopfen zum Aufsetzen auf ein Anschlussmittel einer medizinischen Spritze derart weiter zu entwickeln, dass derartige Vorfälle mit zum Teil letalen Folgen für die Patienten auszuschließen sind.

Aus der IE 911 804 A1 ist zwar ein Stopfen nach dem Oberbegriff das Anspruchs 1 bekannt, dieser ist aber nicht zum Ausbringen eines Wirkstoffes, eines Pharmazeutikums oder einer Nährstofflösung geeignet und liefrt daher keinen Anhaltspunkt dir die Lösung dieser Aufgabe. Gelöst wird diese Aufgabe durch einen Stopfen zum Aufsetzen auf ein Anschlusselement einer medizinischen Spritze mit allen Merkmalen des Patentanspruchs 1 oder 2. Vorteilhafte Ausgestaltungen der Erfindung finden sich in den Unteransprüchen. Ferner wird die Aufgabe gelöst von den Verwendungsansprüchen 11 und 12.

Der erfindungsgemäße Stopfen zum Aufsetzen auf ein Anschlusselement einer medizinischen Spritze, die mit dem Anschlusselement (1) verbindbar ist, zeichnet sich dabei u.a. dadurch aus, dass der Stopfen einen Durchgangskanal aufweist und frei von weiteren Konnektivitäts- oder Verbindungselementen ausgebildet ist. Ein derartiger erfindungsgemäßer Stopfen ist dabei kompatibel zu den gängigen Spritzenanschlüssen ausgebildet, sodass nach dem er auf die Spritze aufgebracht beziehungsweise aufgesetzt wurde, das Aufbringen eines anderen Applikationselementes, wie beispielsweise eines Schlauches, einer Kanüle oder auch eines Katheters verhindert ist. Damit aber weiterhin die geplante orale oder dermale Applikation möglich ist, weist der Stopfen entgegen dem aus dem Stand der Technik bekannten Verschlüssen eine Öffnung aus, durch welche der in der Spritze aufgenommene Wirkstoff beziehungsweise das Pharmazeutikum oder die Ernährungslösung in den Mund oder auch auf die Haut des Patienten appliziert werden kann. Hierbei ist unter einem derartigen Stopfen jedes Aufsetzelement zu verstehen, welches entsprechend eines Verschlusses, wie beispielsweise des eingangs erwähnte TELC-Verschlusses, auf das Anschlusselement aufgesetzt werden kann, und bei dem durch den Durchgangskanal ein in der Spritze aufgenommenes Medium oral oder dermal appliziert werden kann. Dadurch dass der Stopfen frei von weiteren Konnektivitäts- oder Verbindungselementen ausgebildet ist, ist ferner auch ausgeschlossen, dass daran weitere Applikationsvorrichtungen zur intravenösen, trans- oder subkutanen Injektion von Wirkstoffen und Pharmazeutika beziehungsweise Nährstofflösungen, wie beispielsweise Kanülen, Katheter oder dergleichen daran angeschlossen werden können.

Durch die Verwendung des erfindungsgemäßen Stopfens ist es daher sicher vermieden, dass eine zur oralen oder dermalen Applikation vorbereitete Spritze anderweitig, insbesondere zur intravenösen, trans- oder subkutanen Injektion von Wirkstoffen und Pharmazeutika beziehungsweise Nährstofflösungen verwendet wird, da auf den erfindungsgemäßen Stopfen kein weiteres Anschluss- beziehungsweise Applikationselement, insbesondere keine Kanüle, kein Katheter und auch kein Schlauch mehr angeschlossen werden kann. Dies führt erfindungsgemäß dazu, dass intravenöse, trans- und/oder subkutane Fehlapplikationen wirkungsvoll vermieden sind.

Nach einer ersten vorteilhaften Ausgestaltung der Erfindung ist der Stopfen daher zur oralen und/oder dermalen Applikation eines Mediums ausgebildet.

Gemäß der Erfindung weist der Stopfen einen Innenkegel eines Luer-Konus auf, der zum Zusammenwirken mit einem Außenkegel eines Luer-Konus einer medizinischen Spritze ausgebildet ist. Da in Krankenhäusern und ähnlichen medizinischen Einrichtungen standardisierte Spritzen mit derartigen Luer-Konussen verwendet werden, hat es sich als vorteilhaft erwiesen, den erfindungsgemäßen Stopfen derart auszubilden, dass sie mit den standardisierten Spritzen verbindbar sind.

In gleicher Weise hat es sich ebenfalls als vorteilhaft erwiesen, dass der Stopfen ein Außengewinde einer Luer-Lock-Verbindung aufweist, das zum Zusammenwirken mit einem Innengewinde einer Luer-Lock-Verbindung einer medizinischen Spritze ausgebildet ist. Insofern ist es auch in einfacher Weise möglich, den erfindungsgemäßen Stopfen mit standardisierten Spritzen mit Luer-Lock-Systemen zu verbinden.

In einer besonders vorteilhaften Ausgestaltung der Erfindung ist der Stopfen zum irreversiblen Aufsetzen auf eine medizinische Spritze ausgebildet. Hierdurch ist sichergestellt, dass ein einmal auf der Spritze aufgesetzter Stopfen nicht mehr davon abgenommen werden kann. Dies ist insbesondere deshalb vorteilhaft, da auch eine bewusste intravenöse Applikation eines zur oralen oder dermalen Applikation vorgesehenen Wirkstoffs, der in der Spritze aufgenommen ist, ausgeschlossen ist. Der erfindungsgemäße Stopfen kann nämlich nicht mehr entfernt werden, sodass auch kein anderes Anschluss- beziehungsweise Applikationselement in Form einer Kanüle, eines Katheters oder einer Schlauchverbindung daran angeschlossen werden kann.

In einer anderen Ausgestaltung der Erfindung weist der Stopfen dabei einen ersten Abschnitt sowie einen zweiten Abschnitt auf, der mit dem ersten Abschnitt über wenigstens eine Sollbruchstelle lösbar verbunden ist. Hierdurch ist insbesondere bei Verwendung von Luer-Lock-Systemen erreicht, dass der zweite Abschnitt über die Sollbruchstellen bei einem Weiterdrehen von dem ersten Abschnitt gelöst werden kann, sodass der erste Abschnitt als männlicher Teil derart in dem als weiblicher Teil des Luer-Lock-Systems ausgebildeten Anschlusselement der medizinischen Spritze aufgenommen beziehungsweise versenkt ist, dass kein Teil des Gegenelementes aus diesem Anschlusselement herausragt. Somit besteht kein Angriffspunkt, um das Gegenelement aus dem Anschlusselement herauszulösen. Hierdurch ist nochmals die Sicherheit erhöht, dass keine intravenöse Injektion des in der Spritze aufgezogenen und für eine orale oder dermale Applikation vorgesehenen Mediums erfolgen kann, da kein anderes Anschlussbeziehungsweise Applikationselement, wie eine beispielsweise eine Kanüle ein Katheter oder ein Schlauchelement daran angeschlossen werden kann. Die Verwendung solcher Sollbruchstellen ist erfindungsgemäß aber nicht auf von Luer-Lock-System beschränkt, sondern kann auch bei anderen Verbindungsarten wie beispielsweise Luer-Slip-Systemen oder gänzlich anderen Verbindungsarten zum Einsatz kommen.

Alternativ ist es natürlich auch möglich eine derartige irreversible Anordnung eines erfindungsgemäßen Stopfens auf einer Spritze, in anderer Art und Weise zu realisieren. Beispielsweise sei hier erwähnt, dass bei Luer-Lock-Systemen das Außengewinde des Gegenelementes zum Beispiel widerhakenartige Elemente aufweist, welche ein Herausdrehen des erfindungsgemäßen Stopfens verhindern, indem sich diese widerhakenartigen Elemente im Innengewinde des Luer-Lockelementes der Spritze festsetzen. Natürlich sind auch andere Geometrien im Gewindegang denkbar mit denen ein Herausdrehen unmöglich gemacht wäre. Im Falle eines Luer-Slip-Systems könnte der Luer-Konus-Anschluss eine entsprechend eng ausgelegte Passung aufweisen. Systemunabhängig könnten auch Rast- und Gegenrastelemente beziehungsweise Clips- und Gegenclipselemente vorgesehen sein.

In einer weiteren Ausgestaltung der Erfindung ist vorgesehen, dass wenigstens ein Markierungselement vorgesehen ist, dass eine mit dem erfindungsgemäßen Stopfen versehene medizinische Spritze zur oralen oder dermalen Applikation kennzeichnet. Solche Markierungselemente können dabei in unterschiedlicher Art und Weise angeordnet sein. In einer Ausführung kann dies ein schriftlicher Hinweis sein, dass die mit einem erfindungsgemäßen Stopfen versehene Spritze nur zur oralen oder dermalen Applikation verwendet werden darf. Andererseits kann das Markierungselement auch ein Bereich des erfindungsgemäßen Stopfens sein, der mit einer Signalfarbe versehen ist. Auch sind entsprechende Kombinationen verschiedener Markierungselemente denkbar.

Um eine ökonomisch effektive und kostengünstige Herstellung der erfindungsgemäßen Stopfen zu gewährleisten, hat es sich bewährt, dass der Stopfen zumindest teilweise aus einem Kunststoff, beispielsweise einem thermoplastischen Elastomer, aus Gummi oder aus Silikon besteht. Die Herstellung mit derartigen Materialen ist verfahrenstechnisch sehr gut zu handhaben und hat sich insbesondere in der gesamten Medizintechnik auch bewährt.

Nach einer weiteren Ausgestaltung der Erfindung ist es dabei vorgesehen, dass der Durchgangskanal des erfindungsgemäßen Stopfens mittels eines Schließelementes verschließbar ist. Hierdurch ist erreicht, dass der Inhalt einer zur oralen Applikation vorbereiteten und mit einem derartigen Stopfen versehenen Spritze nach dem Aufziehen des Wirkstoffes, des Pharmazeutikums oder der Ernährungslösung durch den Durchgangskanal kontaminiert werden kann. Das Schließelement verschließt den Inhalt der Spritze dabei sicher gegen die Umgebung. Kontaminierungen sind daher ausgeschlossen.

Vorteilhafterweise ist das Schließelement dabei als Ventil oder dergleichen, insbesondere als Rückschlagventil, Kugelrückschlagventil, Tellerrückschlagventil, Rückschlagklappe oder dergleichen ausgebildet. Derartige Ventile bieten den Vorteil, dass sie für Medien nur in eine Richtung durchgängig sind. Nachdem die Spritze zur oralen oder dermalen Applikation vorbereitet und mit dem erfindungsgemäßen Stopfen verschlossen wurde, ist es dann nicht mehr möglich, weitere Medien in die Spritze aufzuziehen, da die beschriebenen Ventile kein Medium mehr in Richtung des Spritzeninhaltes durchlassen. Der Spritzeninhalt wird aber nach Betätigen des Spritzenkolbens aus dem Spritzenraum durch den Durchgangskanal herausgetrieben, wobei die beschriebenen Ventile nun für das Medium in diese Richtung durchlässig sind.

Weitere Ziele Vorteile, Merkmale und Anwendungsmöglichkeiten der vorliegenden Erfindung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1:: ein erstes Ausführungsbeispiel eines erfindungsgemäßen Stopfens in einer Querschnittdarstellung,
- Figur 2:: ein zweites Ausführungsbeispiel eines erfindungsgemäßen Stopfens in einer seitlichen Draufsicht,
- Figur 3:: das Ausführungsbeispiel der Figur 1 in einer Draufsicht mit entsprechendem Luer-Anschluss,
- Figur 4:: das Ausführungsbeispiel der Figur 2 in einer Querschnittdarstellung mit einem entsprechenden Luer-Lock-Anschluss,
- Figur 5:: ein drittes Ausführungsbeispiel eines erfindungsgemäßen Stopfens in zwei unterschiedlichen Zuständen,
- Figur 6:: das Ausführungsbeispiel der Figur 5 in einer Querschnittdarstellung mit einem entsprechenden Luer-Lock-Anschluss in zwei unterschiedlichen Zuständen.

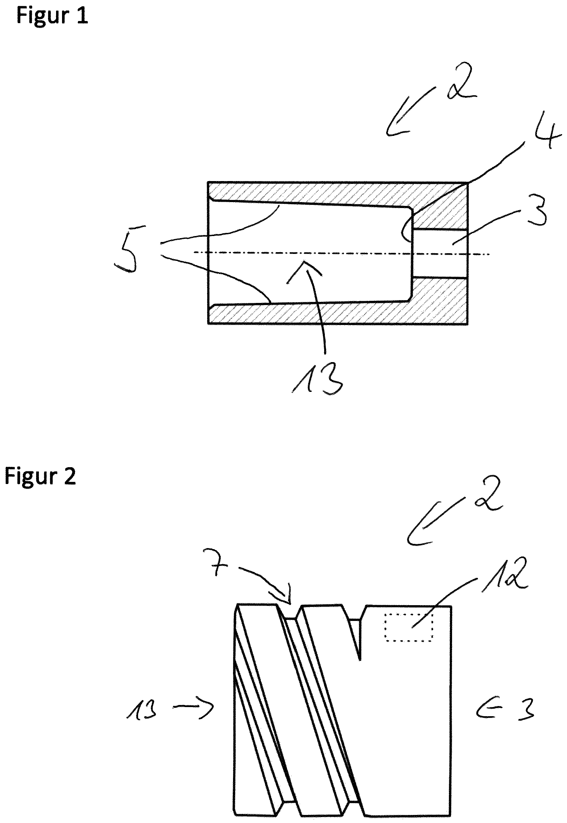

In Figur 1 ist ein erstes Ausführungsbeispiel eines erfindungsgemäßen Stopfens 2 in einer Querschnittdarstellung gezeigt. Der Stopfen 2 weist dabei einen Innenkegel 5 auf, durch den einen Hohlraum 13 gebildet ist. Der Innenkegel 5 läuft dabei von der linken zur rechten Seite konisch zu, sodass sich der Hohlraum 13 zur rechten Seite hin verjüngt. An den Hohlraum 13 schließt sich ein Durchgangskanal 3 an, sodass ein Medium ungehindert durch den Stopfen 2 hindurchtreten kann.

Damit ein Medium nur in eine Richtung durch den Stopfen 2 hindurchtreten kann, kann ein hier nicht dargestelltes Schließelement vorgesehen sein, das vorteilhafterweise als Rückschlagventil, Kugelrückschlagventil, Tellerrückschlagventil, Rückschlagklappe oder dergleichen ausgebildet ist.

In einer hier nicht dargestellten Ausführungsform sind an dem Stopfen 2 noch Rast- oder Clipselemente angebracht. Diese können mit Gegenrast- beziehungsweise Gegenclipselementen eines Anschlusselementes 1 einer Spritze derart zusammenwirken, dass der Stopfen 2 unlösbar und irreversibel an einem Anschlusselement 1 einer Spritze befestigt werden kann.

In einer weiteren hier nicht dargestellten Ausführungsform können bei einem Luer-Lock-System in einem Außengewinde 7 des Stopfens 2 widerhakenartige Elemente vorgesehen sein. Diese widerhakenartigen Elemente verhindern ebenfalls, dass ein einmal auf eine Spritze aufgesetzter Stopfen 2 davon wieder gelöst werden kann.

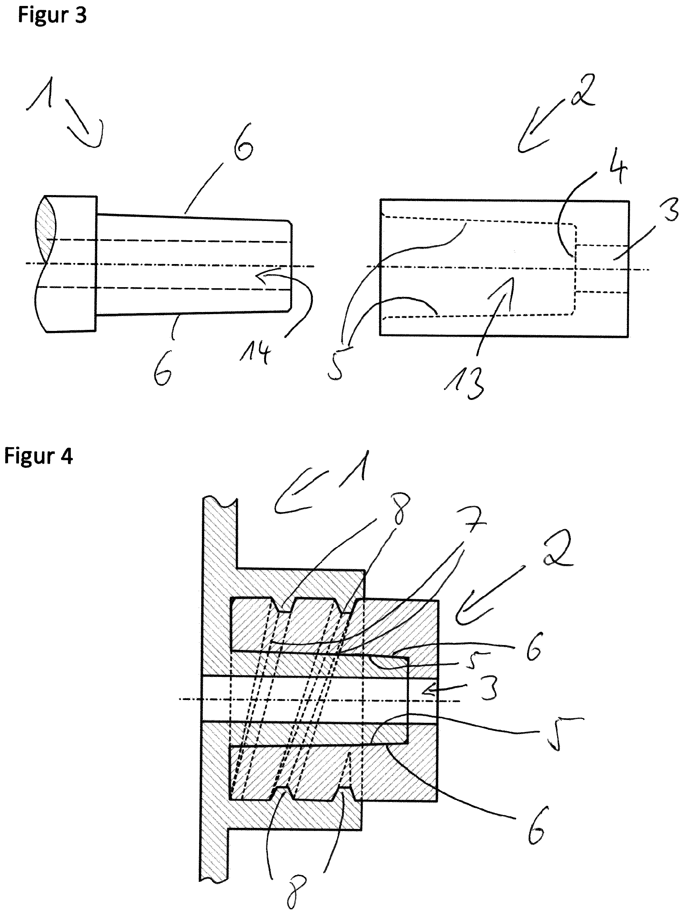

In Figur 3 ist ein derartiges Anschlusselement 1 gezeigt. Wie daraus zu entnehmen ist, weist dieses Anschlusselement 1 einen Außenkegel 6 sowie einen Mediumkanal 14 auf. Wie ebenfalls der Figur 3 zu entnehmen sind der Außenkegel 6 des Anschlusselementes 1 sowie der Innenkegel 5 des Gegenelementes 2 als ein einheitliches Luer-Slip-System ausgebildet, sodass der Innenkegel 5 des Gegenelements 2 als weiblicher Teil dieses Systems den Außenkegel 6 des Anschlusselementes 1 als männlicher Teil aufnimmt und somit eine Verbindung zwischen Anschlusselement 1 und Stopfen 2 sichergestellt ist.

In Figur 2 ist nun ein anderes Ausführungsbeispiel eines erfindungsgemäßen Stopfens 2 in einer Draufsicht gezeigt. Dabei ist zu erkennen, dass der dortige Stopfen 2 zylindrisch ausgebildet und mit einem Außengewinde 7 versehen ist. Ferner ist in der Darstellung der Figur 2 deutlich ein Markierungselement 12 zu erkennen. Diese Markierungselement 12 dient dazu, eine mit dem Stopfen 2 versehene medizinische Spritze zur oralen beziehungsweise dermalen Applikation zu kennzeichnen. Solche Markierungselemente 12 können dabei in unterschiedlicher Art und Weise angeordnet sein. In einer Ausführung kann dies ein schriftlicher Hinweis sein, dass die mit einem erfindungsgemäßen Stopfen 2 versehene Spritze nur zur oralen beziehungsweise dermalen Applikation verwendet werden darf. Andererseits kann das Markierungselement 12 auch ein Bereich des erfindungsgemäßen Stopfens 2 sein, der mit einer Signalfarbe versehen ist, wobei es natürlich auch möglich ist, den gesamten Stopfen 2 mit dieser Signalfarbe zu versehen. Auch sind entsprechende Kombinationen verschiedener Markierungselemente 12 denkbar.

In der Darstellung der Figur 4 ist nun der erfindungsgemäße Stopfen 2 gemäß Figur 2 mit seinem Außengewinde 7 in einem Innengewinde 8 eines Anschlusselementes 1 eingedreht. Insofern bildet das dortige System nun ein Luer-Lock-System. Auch hierbei ist der Stopfen 2 mit einem Hohlraum 13, wie er in Figur 2 angezogen aber in Figur 4 aus Übersichtlichkeitsgründen nicht mit diesem Bezugszeichen versehen ist, ausgebildet, an dem sich entsprechend dem Hohlraum 13 des Ausführungsbeispiels der Figur 1 ein Durchgangskanal 3 anschließt. Die Verbindung zwischen Anschlusselement 1 und Stopfen 2 wird dabei in analoger Weise wie bei dem Ausführungsbeispiel der Figuren 1 und 3 realisiert, wobei durch das Innengewinde 8 und das Außengewinde 7 eine exakte Führung - wie bei Luer-Lock-Systemen üblich - gegeben ist. Durch das Markierungsfeld 12 ist nunmehr dem Anwender angezeigt, dass eine mit einem derartigen Stopfen versehene Spritze nicht mehr mit einem anderen Koppelelement, wie beispielsweise einer Kanüle oder ein Schlauchsystem - versehen beziehungsweise verbunden werden kann und nur noch zur oralen beziehungsweise dermalen Applikation verwendet werden darf.

Auch bei diesem Ausführungsbeispiel kann es vorgesehen sein, was vorliegend aber nicht dargestellt ist, dass eine Irreversibilität der Verbindung von Anschlusselement und Stopfen 2 gegeben ist. Auch hier kann diese Irreversibilität dadurch erreicht werden, dass an dem Gegenelement 2 noch Rast- oder Clipselemente angebracht werden. Diese können mit Gegenrast- beziehungsweise Gegenclipselementen eines Anschlusselementes 1 einer Spritze derart zusammenwirken, dass der Stopfen 2 unlösbar und irreversibel an einem Anschlusselement 1 einer Spritze befestigt werden kann.

Alternativ kann es natürlich auch vorgesehen sein, dass das Außengewinde 7 des Stopfens 2 widerhakenartige Elemente aufweist, welche ein Herausdrehen des erfindungsgemäßen Stopfens 2 verhindern, indem sich diese widerhakenartigen Elemente im Innengewinde 8 des Luer-Lockelementes der Spritze beziehungsweise des Anschlusselementes 1 festsetzen.

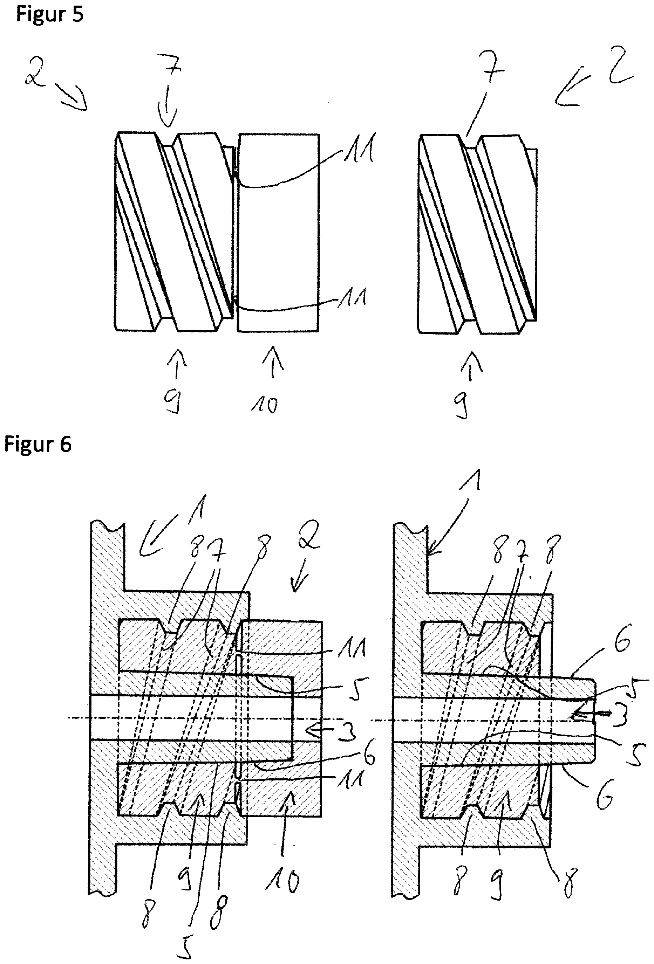

In den Figuren 5 und 6 ist abschließend ein drittes Ausführungsbeispiel eines erfindungsgemäßen Stopfens 2 gezeigt, die in ihrer Grundfunktion dem Ausführungsbeispiel der Figuren 2 und 4 entspricht. Der grundlegende Unterschied zum Ausführungsbeispiel der Figuren 2 und 4 besteht darin, dass der Stopfen 2 einen ersten Abschnitt 9 und einen zweiten Abschnitt 10 aufweist, welche mittels wenigstens einer Sollbruchstelle 11 miteinander verbunden sind.

Dabei kann es vorgesehen sein, dass genau eine Solbruchstelle 11 vorgesehen ist, die das Gegenelement 2 ringförmig umläuft und als eine Materialschwächung ausgebildet ist. Andererseits können auch eine Mehrzahl von Sollbruchstellen 11 vorgesehen sein, die beispielsweise als Stege ausgebildet sind.

Mit dem Stopfen 2 des Ausführungsbeispiels der Figuren 5 und 6 ist es ebenfalls möglich, sicherzustellen, dass eine mit einem solchen Stopfen 2 versehene Spritze nicht mehr zur intravenösen, transkutane oder subkutanen Applikation verwendet werden kann. Der erfindungsgemäße Stopfen 2 dieses Ausführungsbeispiels hat vor dem Aufsetzen auf eine Spritze, die in der linken Darstellung der Figur 5 gezeigte Gestalt. Dieser Stopfen 2 wird nun mit dem Außengewinde 7 in das Innengewinde 8 des Anschlusselementes solange eingedreht, bis der Außenkegel 6 des Anschlusselementes 1 am Innenkegel 5 des Stopfens 2 zum Liegen kommt. Diese Anordnung ist in der linken Darstellung der Figur 6 gezeigt. Dabei sind das Anschlusselement 1 und der Stopfen 2 nun miteinander verbunden.

Damit nun der Stopfen 2 nicht wieder einfach aus dem Anschlusselement 1 der Spritze herausgedreht werden kann und doch wieder eine Kanüle oder dergleichen mit dem Abschlusselement 1 beziehungsweise der Spritze verbunden werden kann, wird der Stopfen 2 in einfacher Weise weiter in Schließrichtung gedreht. Dadurch bricht/brechen die Sollbruchstelle/n 11, sodass sich der zweite Abschnitt 10 der Stopfens 2 vom ersten Abschnitt 9 des Stopfens 2 löst.

Wie aus der rechten Darstellung der Figur 6 zu entnehmen ist, ist dabei der erste Abschnitt 9 des Stopfens 2 nun derart in dem Anschlusselement 1 zwischen dessen Innengewinde 8 und dessen Außenkegel 6 aufgenommen, dass kein Teil des ersten Abschnittes 9 des Stopfens 2 dort herausragt. Insofern besteht auch nicht die Möglichkeit, diesen ersten Abschnitt 9 des Stopfens 2 wieder aus dem Anschlusselement 1 der Spritze herauszudrehen. Hierdurch ist sichergestellt, dass eine mit einem derartigen Stopfen 2 versehene Spritze für eine intravenöse, transkutane oder subkutane Applikation unbrauchbar ist, das an das Anschlusselement 1 der Spritze keine Kanüle, Katheder oder dergleichen mehr anschließbar ist.

Wie insbesondere der rechten Darstellung der Figur 6 zu entnehmen ist, ist das an der Spritze verbleibende Element 9 des Stopfens 2 derart an der Spritze angeordnet, dass es bei einer oralen beziehungsweise dermalen Applikation auch beim Applizieren in der Regel nicht in Kontakt mit dem zu applizierenden Medium kommt. Das Medium gelangt dabei bei sachgerechter Anwendung nur in Kontakt mit einem in dem Anschlusselement 1 angeordneten Mediumkanal, wie er in den Figuren 4 und 6 dargestellt ist.

### Bezugszeichenliste

- 1: Anschlusselement
- 2: Stopfen
- 3: Durchgangskanal
- 5: Innenkegel
- 6: Außenkegel
- 7: Außengewinde
- 8: Innengewinde
- 9: erster Abschnitt
- 10: zweiter Abschnitt
- 11: Sollbruchstelle
- 12: Markierungselement
- 13: Hohlraum
- 14: Mediumkanal

## Patentansprüche

1. Stopfen (2) zum Aufsetzen auf ein Anschlusselement (1) einer medizinischen Spritze, wobei das Anschlusselement (1) mit der medizinischen Spritze verbunden ist und einen Außenkegel (6) eines Luer-Konus sowie einen Mediumkanal (14) aufweist,
**dadurch gekennzeichnet, dass**
der Stopfen (2) einen Innenkegel (6) aufweist, in welchem der Außenkegel (6) des Anschlusselements (1) aufgenommen ist, der Stopfen (2) einen Durchgangskanal (3), durch welchen ein in der Spritze enthaltenes Medium in Form eines Wirkstoffes, eines Pharmazeutikums oder einer Nährstofflösung hindurchtritt, aufweist und frei von weiteren Konnektivitäts- oder Verbindungselementen ausgebildet ist, wodurch der Stopfen (2) mit keinem weiteren Anschluss- oder Applikationselement verbindbar ist, wobei der Innenkegel (5) des Stopfens (2) einen Luer-Konus aufweist, der zum Zusammenwirken mit einem Außenkegel (6) eines Luer-Konus der medizinischen Spritze ausgebildet ist, wobei der Stopfen kreiszylindrisch ausgestaltet ist und eine plane distale Frontfläche aufweist.

2. Stopfen (2) zum Aufsetzen auf ein Anschlusselement (1) einer medizinischen Spritze, wobei das Anschlusselement (1) mit der medizinischen Spritze verbunden ist und einen Außenkegel (6) eines Luer Konus sowie einen Mediumkanal (14) aufweist,
**dadurch gekennzeichnet, dass**
der Stopfen (2) einen Innenkegel (6) eines Luer Konus aufweist, in welchem der Außenkegel (6) des Anschlusselements (1) aufgenommen ist, wobei der Stopfen (2) einen Durchgangskanal (3), durch welchen ein in der Spritze enthaltenes Medium in Form eines Wirkstoffes, eines Pharmazeutikums oder einer Nährstofflösung hindurchtritt, aufweist und frei von weiteren Konnektivitäts- oder Verbindungselementen ausgebildet ist, wodurch der Stopfen (2) mit keinem weiteren Anschluss- oder Applikationselement verbindbar ist, wobei der Stopfen (2) ein Außengewinde (7) einer Luer-Lock-Verbindung aufweist, das zum Zusammenwirken mit einem Innengewinde (8) einer Luer-Lock-Verbindung einer medizinischen Spritze ausgebildet ist, wobei der Stopfen kreiszylindrisch ausgestaltet ist und eine plane distale Frontfläche aufweist..

3. Stopfen nach einem der vorhergehenden Ansprüche,
**dadurch gekennzeichnet, dass**
der Stopfen (2) Rast- oder Clipselemente aufweist, welche mit Gegenrast- oder Gegenclipselementen des Anschlusselementes (1) zusammenwirken, so dass der Stopfen (2) irreversibel an dem Anschlusselement (1) befestigbar ist.

4. Stopfen nach einem der vorhergehenden Ansprüche,
**dadurch gekennzeichnet, dass**
der Stopfen (2) widerhakenartige Elemente aufweist, mit welchen der Stopfen (2) irreversibel an dem Anschlusselement (1) befestigbar ist.

5. Stopfen nach einem der vorhergehenden Ansprüche,
**dadurch gekennzeichnet, dass**
der Stopfen (2) einen ersten Abschnitt (9) sowie einen zweiten Abschnitt (10) aufweist, der mit dem ersten Abschnitt (9) über wenigstens eine Sollbruchstelle (11) lösbar verbunden ist.

6. Stopfen nach Anspruch 5,
**dadurch gekennzeichnet, dass**
die wenigstens eine Sollbruchstelle (11) als Steg ausgebildet ist.

7. Stopfen nach Anspruch 5 oder 6,
**dadurch gekennzeichnet, dass**
der ersten Abschnitt (9) derart ausgebildet ist, dass er im verbunden Zustand des Stopfens (2) mit der Spritze in dem Anschlusselement ganz aufgenommen und/oder versenkt ist.

8. Stopfen nach einem der vorhergehenden Ansprüche,
**dadurch gekennzeichnet, dass**
wenigstens ein Markierungselement (12) vorgesehen ist, das eine mit dem Stopfen (2) versehene medizinischen Spritze für eine bestimmte Applikationsart kennzeichnet.

9. Stopfen nach einem der vorhergehenden Ansprüche
**dadurch gekennzeichnet, dass**
der Stopfen (2) zumindest teilweise aus einem Kunststoff, beispielsweise einem thermoplastischen Elastomer, aus Gummi oder aus Silikon besteht.

10. Stopfen nach einem der vorhergehenden Ansprüche,
**dadurch gekennzeichnet, dass**
ein Schließelement vorgesehen ist, welches als Ventil, insbesondere als Rückschlagventil, Kugelrückschlagventil, Tellerrückschlagventil, Rückschlagklappe oder dergleichen ausgebildet ist, sodass ein Medium den Durchgangskanal (3) nur in Richtung aus der medizinischen Spritze hinaus passieren kann.

## Claims

1. Plug (2) for mounting on a connecting element (1) of a medical syringe, the connecting element (1) being connected to the medical syringe and having an outer taper (6) of a Luer cone and a medium channel (14),
**characterised in that**
the plug (2) has an inner taper (6) in which the outer taper (6) of the connecting element (1) is received, the plug (2) has a through-channel (3) through which a medium contained in the syringe in the form of an active ingredient, a pharmaceutical or a nutrient solution passes, and is designed to be free of any further connectivity or connecting elements, as a result of which the plug (2) cannot be connected to any further connecting or application element, the inner taper (5) of the plug (2) having a Luer cone which is designed to interact with an outer taper (6) of a Luer cone of the medical syringe, the plug being circular cylindrical and having a flat distal front face.

2. Plug (2) for mounting on a connecting element (1) of a medical syringe, the connecting element (1) being connected to the medical syringe and having an outer taper (6) of a Luer cone and a medium channel (14),
**characterised in that**
the plug (2) has an inner taper (6) of a Luer cone in which the outer taper (6) of the connecting element (1) is received, the plug (2) having a through-channel (3) through which a medium contained in the syringe in the form of an active ingredient, a pharmaceutical or a nutrient solution passes, and is designed to be free of any further connectivity or connecting elements, as a result of which the plug (2) cannot be connected to any further connecting or application element, the plug (2) having an outer thread (7) of a Luer lock connection, which is designed to interact with an inner thread (8) of a Luer lock connection of a medical syringe, the plug being circular cylindrical and having a flat distal front face.

3. Plug according to any of the preceding claims,
**characterised in that**
the plug (2) has latching or clip elements which interact with mating latching elements or mating clip elements of the connecting element (1) such that the plug (2) is non-reversibly attachable to the connecting element (1).

4. Plug according to any of the preceding claims,
**characterised in that**
the plug (2) has barb-like elements by means of which the plug (2) is non-reversibly attachable to the connecting element (1).

5. Plug according to any of the preceding claims,
**characterised in that**
the plug (2) has a first portion (9) and a second portion (10) which is detachably connected to the first portion (9) via at least one predetermined breaking point (11).

6. Plug according to claim 5,
**characterised in that**
the at least one predetermined breaking point (11) is designed as a neck.

7. Plug according to either claim 5 or claim 6,
**characterised in that**
the first portion (9) is designed such that, in the connected state of the plug (2) to the syringe, it is completely received and/or sunk in the connecting element.

8. Plug according to any of the preceding claims,
**characterised in that**
at least one marker element (12) is provided which designates a medical syringe provided with the plug (2) for a particular type of application.

9. Plug according to any of the preceding claims,
**characterised in that**
the plug (2) consists at least in part of a plastics material, for example a thermoplastic elastomer, of rubber or of silicone.

10. Plug according to any of the preceding claims,
**characterised in that**
a closing element is provided which is designed as a valve, in particular as a check valve, ball check valve, lift check valve, clack valve or the like, such that a medium can pass through the through-channel (3) only in the direction that leads out of the medical syringe.

## Revendications

1. Bouchon (2) à placer sur un élément de raccordement (1) d'une seringue médicale, l'élément de raccordement (1) étant relié à la seringue médicale et présentant un cône externe (6) d'un cône Luer ainsi qu'un canal de milieu (14),
**caractérisé par le fait que**
le bouchon (2) présente un cône interne (6) dans lequel le cône externe (6) de l'élément de raccordement (1) est reçu, le bouchon (2) présente un canal de passage (3) à travers lequel passe un milieu contenu dans la seringue sous la forme d'un principe actif, d'un produit pharmaceutique ou d'une solution nutritive, et est formé sans autres éléments de connexion ou de liaison, ce par quoi le bouchon (2) est apte à être relié à aucun autre élément de raccordement ou d'application, le cône interne (5) du bouchon (2) présentant un cône Luer, qui est formé pour coopérer avec un cône externe (6) d'un cône Luer de la seringue médicale, le bouchon étant réalisé en forme de cylindre circulaire et présentant une surface frontale distale plane.

2. Bouchon (2) à placer sur un élément de raccordement (1) d'une seringue médicale, l'élément de raccordement (1) étant relié à la seringue médicale et comprenant un cône externe (6) d'un cône Luer ainsi qu'un canal de milieu (14),
**caractérisé par le fait que**
le bouchon (2) présente un cône interne (6) d'un cône Luer dans lequel le cône externe (6) de l'élément de raccordement (1) est reçu, le bouchon (2) présentant un canal de passage (3) à travers lequel passe un milieu contenu dans la seringue sous la forme d'un principe actif, d'un produit pharmaceutique ou d'une solution nutritive, et étant formé sans autres éléments de connexion ou de liaison, ce par quoi le bouchon (2) est apte à être relié à aucun autre élément de raccordement ou d'application, le bouchon (2) présentant un filetage externe (7) d'un raccord Luer-Lock, qui est formé pour coopérer avec un filetage interne (8) d'un raccord Luer-Lock d'une seringue médicale, le bouchon (2) étant réalisé en forme de cylindre circulaire et présentant une surface frontale distale plane.

3. Bouchon selon l'une des revendications précédentes,
**caractérisé par le fait que**
le bouchon (2) présente des éléments d'encliquetage ou de clipsage, lesquels coopèrent avec des contre-éléments d'encliquetage ou de clipsage de l'élément de raccordement (1), de telle sorte que le bouchon (2) est apte à être fixé à l'élément de raccordement (1) de manière irréversible.

4. Bouchon selon l'une des revendications précédentes,
**caractérisé par le fait que**
le bouchon (2) présente des éléments en forme de barbillons, au moyen desquels le bouchon (2) est apte à être fixé à l'élément de raccordement (1) de manière irréversible.

5. Bouchon selon l'une des revendications précédentes,
**caractérisé par le fait que**
le bouchon (2) présente une première partie (9) ainsi qu'une deuxième partie (10) qui est reliée à la première partie (9) de manière amovible au moyen d'au moins une zone de rupture (11).

6. Bouchon selon la revendication 5,
**caractérisé par le fait que**
ladite au moins une zone de rupture (11) est formée en tant que barre.

7. Bouchon selon l'une des revendications 5 ou 6,
**caractérisé par le fait que**
la première partie (9) est formée de telle sorte qu'elle est complètement reçue et/ou enfoncée dans l'élément de raccordement dans l'état relié du bouchon (2) avec la seringue.

8. Bouchon selon l'une des revendications précédentes,
**caractérisé par le fait que**
il est prévu au moins un élément de marquage (12) qui identifie une seringue médicale munie du bouchon (2) pour un type d'application déterminé.

9. Bouchon selon l'une des revendications précédentes,
**caractérisé par le fait que**
le bouchon (2) est constitué au moins partiellement d'une matière plastique, par exemple d'un élastomère thermoplastique, de caoutchouc ou de silicone.

10. Bouchon selon l'une des revendications précédentes,
**caractérisé par le fait que**
il est prévu un élément de fermeture, lequel est formé en tant que soupape, en particulier en tant que soupape anti-retour, soupape anti-retour à bille, soupape anti-retour à plateau, clapet anti-retour ou similaires, de telle sorte qu'un milieu puisse passer à travers le canal de passage (3) uniquement dans une direction hors de la seringue médicale.
